(19) Europäisches Patentamt
European Patent Office
Office européen des brevets

(11) EP 3 766 676 A1

(12) EUROPEAN PATENT APPLICATION

(43) Date of publication:
**20.01.2021 Bulletin 2021/03**

(21) Application number: **19195034.4**

(22) Date of filing: **03.09.2019**

(51) Int Cl.:
***B29C 70/48*** *(2006.01)* ***C08G 18/67*** *(2006.01)*
***C08J 5/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.07.2019 CN 201910650931**

(71) Applicant: **Covestro Deutschland AG**
**51373 Leverkusen (DE)**

(72) Inventors:
- **ZHENG, Ian**
**Zhujing Town**
**201500 Jinshan District**
**Shanghai (CN)**
- **GU, Yongming**
**201208 Pudong New District**
**Shanghai (CN)**
- **WU, Di**
**201206 Pudong**
**Shanghai (CN)**

(74) Representative: **Levpat**
**c/o Covestro AG**
**Gebäude 4825**
**51365 Leverkusen (DE)**

# (54) METHOD FOR PREPARING A POLYURETHANE COMPOSITE BY A VACUUM INFUSION PROCESS

(57) The invention relates to a method for preparing a polyurethane composite by a vacuum infusion process, a polyurethane composite obtained by the method and use thereof. The method of the present invention for preparing a polyurethane composite by a vacuum infusion process makes is possible to determine the time for infusing polyurethane compositions after completing the dehumidification easily and intuitively, thereby improving production efficiency and saving production costs.

Fig. 1

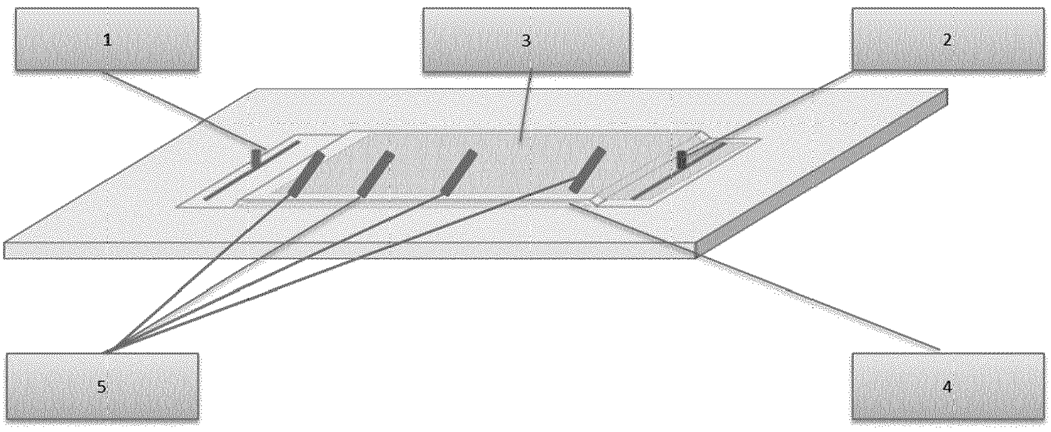


EP 3 766 676 A1

## Description

### Technical field

The invention relates to a method for preparing a polyurethane composite by a vacuum infusion process, a composite obtained by the method and use thereof.

### Prior art

In recent years, the superiority of polyurethane composites in the production of wind turbine blades has received increasing attention. Wind energy is considered to be one of the cleanest and most environmentally friendly energy sources available today. So wind turbines have always been demanded by the market. Compared with conventional wind turbine blades made from epoxy resins, wind turbine blades made from polyurethane composites have the advantages of lower cost and better physical properties. However, polyurethane is sensitive to water, while core materials, reinforcing materials, flow media, etc. used to produce the wind turbine blades usually contain a certain amount of moisture. Thus, it is necessary to carry out dehydration by drying before infusing polyurethane compositions. Moreover, it is necessary in the vacuum infusion to lay films. Because a lot of layers exist under the film and the large parts are bulky, the overall moisture content in the layers cannot be detected by conventional methods such as weighing. Therefore, detecting the degree of drying/dehydration for meeting the requirements is at present time-consuming and depends on experience. No objective indicators or methods can be used for characterization. How to objectively indicate whether the drying is completed and whether the infusion of the polyurethane resin/composition can be started is an urgent problem to be solved in the industry.

CN108152276A discloses a test piece and a test method for testing surface moisture of a tinned steel plate. The technical problem to be solved is the difficulty in detecting the surface moisture of an existing cold-rolled tinned steel plate and the corrosion to the cold-rolled tinned steel plate in the detection process. The technical solution is a test piece for testing the surface moisture of the tinned steel plate, a test piece for testing the surface moisture of the tinned steel plate, wherein the test piece is blue and comprises a polyurethane foam plastic sheet soaked with a detection reagent. The test piece is characterized in that the detection reagent is prepared from the following components in percentage by volume: 0.1 to 20 percent of chloride, 0.05 to 5 percent of surfactant and the rest of water. The polyurethane foam plastic sheet soaked with the detection reagent is squeezed and then dried at a temperature of 105 to 125°C for 20 to 40 minutes by using an oven to obtain a blue test piece. The test piece disclosed by the literature is simple to produce and easy to operate, has no corrosion to tinned steel plates, has a high detection speed and a high sensitivity.

CN103770341B discloses a processing system for a carbon fiber reinforced composite and a controllable carbon fiber self-heating method using a liquid molding technology. The processing system comprises a vacuum bag encapsulation system, a positive foil electrode, a negative foil electrode, a low-voltage regulator, a program controlled temperature regulator and a temperature sensor. The positive foil electrode and the negative foil electrode are arranged at two ends of a continuous carbon fiber, wherein the continuous carbon fiber is arranged in a vacuum bag. The low-voltage regulator supplies heat to the continuous carbon fiber, wherein the heat is controlled by the program controlled temperature regulator. The quick carbon fiber self-heating method using the liquid molding technology disclosed by the invention can realize quick-heating of carbon fiber layers, and provide a program controllable quick-heating method for a carbon fiber layers for the liquid molding technology and other molding technologies for carbon fiber reinforced composites. Thus, the heating time and cooling time in a composite molding process are greatly shortened, and a technical support is provided for quick molding of the carbon fiber reinforced composites.

US8707762 describes a low-cost, easily manufactured humidity and mold indicator for buildings that incorporates a one-piece transparent plastic injection-molded body. Appearance of a warning icon or a change in color alerts the owners or occupants of a building to a hidden leak and thereby prevents costly structural damage, cosmetic damage and mold damage to building components. The indicator is easily inserted and secured into a drilled hole in panel such as a wall or soffit. In one example, the indicator snaps into place after being fully inserted into the panel. In another example, a retainer inserted into the hole holds the indicator enabling easier removal of the indicator for replacement or for inspection of the area behind the panel. An optional removable cap can be painted the same color as the wall. An optional mask covers the integral viewing window during painting of the device.

US2017003229 A1 discloses a system and method for determining the presence of water proximate to insulation. The system provided includes an enclosure, and insulation disposed over the enclosure. A moisture reactive compound indicates water proximate to the insulation.

Despite the above disclosure, there is an urgent need for a more efficient and superior method for preparing polyurethane composites.

**Summary of the invention**

One aspect of the present invention is to provide a method for preparing a polyurethane composite by a vacuum infusion process, comprising:

placing at least a reinforcing material, a core material and/or a flow medium on a mold, and placing at least a humidity/moisture indicating paper/agent on the reinforcing material, the core material or the flow medium;
dehumidifing under vacuum until the humidity/moisture indicating paper/agent changes color;
introducing a polyurethane composition, and demolding after curing to obtain the polyurethane composite.

Preferably, the humidity/moisture indicating paper/agent is selected from the group consisting of cobalt chloride, and the color is changed to blue. More preferably, the color is changed to any color selected from the group consisting of those with range b* < -15, preferably < -17, more preferably - 25 to -18 in color values (according to test method CIE 1976 L*a*b*). The color values are measured according to the CIE 1976 L*a*b* standard using a spectro-guide 45/0 gloss colorimeter provided by BYK Corporation for example.
Preferably, the color is changed to any color selected from the group consisting of those with range L* ≤ 80, preferably ≤ 75, more preferably ≤ 73; a* < -5, preferably < -7, more preferably < -8 in color values (according to test method CIE 1976 L*a*b*).
Preferably, the dehumidification comprises heating, wherein the heating temperature is firstly set to 40 to 50 °C, preferably 40 to 45 °C, and then gradually reduce to 20 to 38 °C.
Optionally, the step b) further comprises:

covering said at least a reinforcing material, a core material and/or a flow medium with a first film, and sealing the rim of the first film with the mold, and vacuumizing between the first film and the mold;
laying a second film to cover the first film, fixing the second film, sealing the rims of the first film and the second film, wherein an air inlet channel and an air outlet channel are reserved;
heating the mold, while filling hot air between the first film and the second film to provide a temperature close to the mold temperature for the upper surface of the first film.

Preferably, the dehumidification comprises heating, and the heating is one, two or more selected from the group consisting of electric blanket heating, electric film heating, microwave heating, infrared heating and hot air heating.
Preferably, the reinforcing material is selected from the group consisting of entangled glass fiber layers, glass fiber woven fabrics and glass fiber gauzes, cut or ground glass fibers or mineral fibers, as well as fiber mats, fiber nonwovens and fiber knits based on polymer fibers, mineral fibers, carbon fibers, glass fibers or aramid fibers and mixtures thereof, more preferably glass fiber mats, glass fiber nonwovens, glass fiber woven fabrics and mixtures thereof.
Preferably, the core material is preferably balsa wood, PVC foam, SAN foam, polyurethane foam, PS foam, PMI foam and PET foam.
Preferably, the flow medium comprises a peel ply, and said peel ply is preferably a polyester peel ply.
Preferably, the polyurethane composition comprises the following components:

a component A, comprising one or more organic polyisocyanates;
a component B, comprising:

b1) one or more organic polyols, which is present in a content of 21 to 60 wt%, based on the total weight of the polyurethane composition as 100 wt%;
b2) one or more compounds having the structure of formula (I)

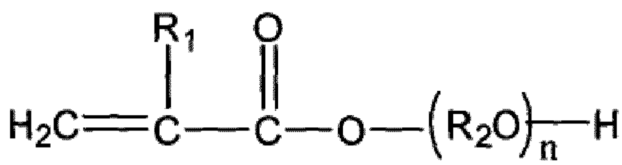

I

wherein R1 is selected from hydrogen, methyl or ethyl; R2 is selected from alkylene groups having 2 to 6 carbon

atoms, 2,2-di(4-phenylene)-propane, 1,4-xylylene, 1,3-xylylene, 1,2-xylylene; n is an integer selected from 1 to 6; and

a component C, a free radical initiator.

Preferably, the organic polyol has a functionality of 1.7 to 6, preferably 1.9 to 4.5 and a hydroxyl value of 150 to 1100 mg KOH/g, preferably 150 to 550 mg KOH/g.

Preferably, the component b2) is present in a content of 4.6 to 33 wt% based on the total weight of the polyurethane composition as 100 wt%

Preferably, the component b2) is one, two or more selected from the group consisting of hydroxyethyl methacrylate, hydroxypropyl methacrylate, hydroxybutyl methacrylate, hydroxyethyl acrylate, hydroxypropyl acrylate and hydroxybutyl acrylate.

Preferably, the method further comprises:

providing a reaction injection device (40), which comprises at least two storage tanks (48, 49) for accommodating the components of the polyurethane composition, a vacuumizing device (50) and feeding units (44a, 44b), wherein each of the feeding units (44a, 44b) is connected to the storage tank (48, 49) via a feeding line (41, 42) and a mixing unit (43), the components from the feeding units (44a, 44b) are mixed together;
wherein the rim of the mold is sealed and the mold is connected to at least an injection line (31), which can be used for vacuumizing the mold (55) and supplying the mixed components to the mold (55); and the mold comprises optionally a drying channel (32) for providing a drying gas (33); during the vacuum infusion, the drying gas is supplied to the mold to dry the core material, the reinforcing material and/or the flow medium (21) placed in the mold; and the mold (55) is vacuumized by means of a vacuum source (34), and the mold is connected to the reaction injection device (40) via the injection line (31) and the injection line (45); and the mold can be vacuumized via the injection line (45) through a laterally closable outlet (46), which is connected to a vacuum source (47);
drying the mold (55) and the reinforcing material, core material and/or flow medium (21) contained therein, the injection line (45), and the optional feeding unit (44a, 44b)/mixing unit (43), wherein optionally the drying gas (33) can be introduced via the drying channel (32);
beginning the vacuum infusion process with introducing the degassed components in the feeding lines (41, 42) from the storage tanks (48, 49) into the feeding units (44a, 44b) of the reaction injection device (40), and obtaining the polyurethane composition from the components in the mixing unit (43), wherein the outlet (46) of the vacuum source (47) is closed before the polyurethane composition arrives;
injecting the polyurethane composition into the mold (55) via the injection line (31) while vacuumizing the mold (55) via the drying channel (32) by the vacuum source (34), wherein the injection pressure measured at the injection port of the injection line (31) is kept lower than the external atmospheric pressure.

The polyurethane resin of the present invention has a viscosity which is greatly reduced compared with epoxy resins, and has good weather resistance and fatigue resistance, and can ensure the service life of the composite articles. The polyurethane composition of the present invention has a short curing cycle, which can improve the utilization rate of devices, and make the resin residue amount to be easily controlled during the production process, which can reduce the production costs. The polyurethane composition of the present invention is non-foamed, does not contain a foaming agent, and cannot even contain water. Since the polyurethane composition reacts with moisture and may be foamed, it needs to be dried during application in the composite. Moreover, the humidity of the production environment of large composite articles is difficult to control, and the material cannot be dried beforehand. In addition, since vacuum infusion typically requires a film/bag film to be applied on the layers in the mold, it is difficult to determine the exact time/degree of completion of drying inside.

Through repeated experiments, we have unexpectedly discovered that the method of the present invention can promptly, objectively and effectively alert the humidity change inside the mold, and can accurately indicate the completion of drying. In other words, the time for starting the infusion of the polyurethane composition can be determined objectively and accurately, thereby increasing production efficiency, saving resources, and being more environmentally friendly. Moreover, the addition of humidity/moisture indicating agent/paper can help determining whether the film/bag film is partially damaged or leaked, so that it can be repaired in time to avoid delays and losses.

In addition, the polyurethane composition contained in the method of the present invention has a longer pot life, so that polyurethane composites having uniform quality and excellent physical properties can be obtained when preparing a large polyurethane product. In particular, for large polyurethane products, the method of the present invention provides an effective solution to the harsh application conditions of polyurethanes (e.g., sensitive to water), achieves high production efficiency while saving costs and being more environmentally friendly.

Still another aspect of the present invention is to provide a polyurethane composite obtained by the method of

the present invention for preparing a polyurethane composite by a vacuum infusion process.

Yet another aspect of the present invention is to provide use of the polyurethane composite of the present invention in a wind turbine blade.

Still another aspect of the present invention is to provide a polyurethane product. It includes a polyurethane composite prepared by a method for preparing a polyurethane composite by a vacuum infusion process.

Preferably, the polyurethane product is selected from the group consisting of a wind turbine blade, a radome, a ship part, a rail, a single or sandwich sheet, preferably a spar cap, a web plate, a blade root and/or a blade housing of a wind turbine blade.

Still another aspect of the present invention is to provide a method for detecting the degree of dehumidification in a polyurethane vacuum infusion process, comprising:

obtaining the color information of a humidity/moisture indicating paper/agent to be detected;
comparing the color information of the humidity/moisture indicating paper/agent to be detected with the target color information, until the color information of the humidity/moisture indicating paper/agent to be detected conforms to the target color information.

Preferably, the detection method is characterized in that it further comprises:

storing the target color information beforehand;
wherein the color information is measured by a color detecting instrument.

More preferably, the color information is a color value measured by spectro-guide 45/0 gloss colorimeter provided by BYK Corporation. Alternatively, the current color of the humidity/moisture of the indicating paper/agent is photographed by a mobile terminal and the color RGB value is obtained.

Still another aspect of the present invention is to provide a computer device comprising:

a database unit, which stores multiple pieces of target color information of detected humidity/moisture indicating paper/agents that achieve the dehumidification standard of a polyurethane vacuum infusion process;
a color comparing unit, which is configured to compare the color information of the humidity/moisture indicating paper/agent to be detected with the target color information, so that it can be determined whether the color information of the humidity/moisture indicating paper/agent conforms to the target color information;
an output unit for detection results, which is configured to output a detection result based on the result of the comparison.

Still another aspect of the present invention is to provide a storage medium having a computer program stored thereon, wherein the computer program is executed by a processor to implement the method of the present invention for detecting the degree of dehumidification in a polyurethane vacuum infusion process.

The method of the present invention for detecting the degree of dehumidification in a polyurethane vacuum infusion process makes it possible to detect the color change conveniently, quickly and accurately by using computer devices, rather than subjectively and difficultly by human eyes. Thus, more objective and accurate detection of the degree of dehumidification is achieved, improving the efficiency of the polyurethane vacuum infusion process and ensuring the preparation of polyurethane composites with better quality. In particular, for the preparation of large polyurethane products such as wind blades, the dehumidification effects can be detected from various angles without requiring many operators to monitor on site, thereby saving human resources and being more economical and environmentally friendly.

**Description of drawings**

The present invention will now be illustrated with reference to the accompanying drawings in which:

Figure 1 shows a mold and layers arranged thereon in the method for preparing a polyurethane composite according to examples of the present invention, wherein 1 represents a suction line; 2 represents a peel ply and a flow mesh; 3 represents an injection line; 4 represents a core material, a fiber reinforcing material; and 5 represents a humidity indicating paper.

The left picture in Figure 2 is a photo showing the surface condition of a polyurethane composite obtained by the method for preparing a polyurethane composite according to Example 1 of the present invention; and the right picture in Figure 2 is a photo showing the surface condition of a polyurethane composite obtained by the method

for preparing a polyurethane composite according to Comparative Example 1.

Figure 3 shows a reaction injection device and a mold of the present invention, wherein 55 represents a mold; 21 represents a core material, a reinforcing material and/or a flow medium; 31 represents an injection line; 32 represents a drying channel; 33 represents dry air; 40 represents a reaction injection device; 41, 42 represents a feeding line; 43 represents a mixing unit; 44a, 44b represents a feeding unit; 45 represents an injection line; 46 represents a closable outlet; 47 represents a vacuum source; 48, 49 represents a storage tank; 50 represents a vacuumizing device.

Figure 4 is a schematic flow diagram S100 showing an embodiment of the method of the present invention for detecting the degree of dehumidification in a polyurethane vacuum infusion process.

Figure 5 shows a schematic block diagram of an embodiment 100 of a computer device of the present invention.

Figure 6 shows a schematic block diagram of another embodiment 200 of a computer device of the present invention.

**Embodiments**

Various aspects of the invention are now described in detail.

One aspect of the invention is to provide a method for preparing a polyurethane composite by a vacuum infusion process, comprising:

placing at least a reinforcing material, a core material and/or a flow medium on a mold, and placing at least a humidity/moisture indicating paper/agent on the reinforcing material, the core material or the flow medium;
dehumidifing under vacuum until the humidity/moisture indicating paper/agent changes color;
introducing a polyurethane composition, and demolding after curing to obtain the polyurethane composite.

Preferably, the humidity/moisture indicating paper/agent is selected from the group consisting of cobalt chloride, and the color is changed to any color selected from the group consisting of those with range b* < -15, preferably < -17, more preferably -25 to -18 in color values (according to test method CIE 1976 L*a*b*). The color values are measured according to the CIE 1976 L*a*b* standard using a spectro-guide 45/0 gloss colorimeter provided by BYK Corporation for example.

Preferably, the color is changed to any color selected from the group consisting of those with range L* ≤ 80, preferably ≤ 75, more preferably ≤ 73; a* < -5, preferably < -7, more preferably < -8 in color values (according to test method CIE 1976 L*a*b*).

Preferably, the dehumidification comprises heating, wherein the heating temperature is firstly set to 40 to 50 °C, preferably 40 to 45 °C, and then gradually reduce to 20 to 38 °C.

Optionally, the step b) further comprises:

covering said at least a reinforcing material, a core material and/or a flow medium with a first film, and sealing the rim of the first film with the mold, and vacuumizing between the first film and the mold;
laying a second film to cover the first film, fixing the second film, sealing the rims of the first film and the second film, wherein an air inlet channel and an air outlet channel are reserved;
heating the mold, while filling hot air between the first film and the second film to provide a temperature close to the mold temperature for the upper surface of the first film.

Preferably, the dehumidification comprises heating, and the heating is one, two or more selected from the group consisting of electric blanket heating, electric film heating, microwave heating, infrared heating and hot air heating.

Preferably, the reinforcing material is selected from the group consisting of entangled glass fiber layers, glass fiber woven fabrics and glass fiber gauzes, cut or ground glass fibers or mineral fibers, as well as fiber mats, fiber nonwovens and fiber knits based on polymer fibers, mineral fibers, carbon fibers, glass fibers or aramid fibers and mixtures thereof, more preferably glass fiber mats, glass fiber nonwovens, glass fiber woven fabrics and mixtures thereof.

Preferably, the core material is preferably balsa wood, PVC foam, SAN foam, polyurethane foam, PS foam, PMI foam and PET foam.

Preferably, the flow medium comprises a peel ply, and said peel ply is preferably a polyester peel ply.

Preferably, the polyurethane composition comprises the following components:

a component A, comprising one or more organic polyisocyanates;

a component B, comprising:

b1) one or more organic polyols, which is present in a content of 21 to 60 wt%, based on the total weight of the polyurethane composition as 100 wt%;
b2) one or more compounds having the structure of formula (I)

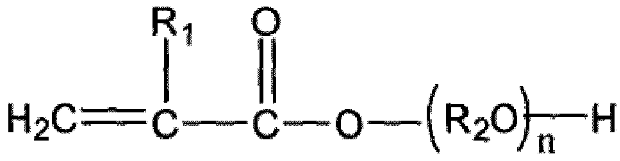

I

wherein R1 is selected from hydrogen, methyl or ethyl; R2 is selected from alkylene groups having 2 to 6 carbon atoms, 2,2-di(4-phenylene)-propane, 1,4-xylylene, 1,3-xylylene, 1,2-xylylene; n is an integer selected from 1 to 6; and

a component C, a free radical initiator.

Preferably, the organic polyol has a functionality of 1.7 to 6, preferably 1.9 to 4.5 and a hydroxyl value of 150 to 1100 mg KOH/g, preferably 150 to 550 mg KOH/g.
Preferably, the component b2) is present in a content of 4.6 to 33 wt% based on the total weight of the polyurethane composition as 100 wt%
Preferably, the component b2) is one, two or more selected from the group consisting of hydroxyethyl methacrylate, hydroxypropyl methacrylate, hydroxybutyl methacrylate, hydroxyethyl acrylate, hydroxypropyl acrylate and hydroxybutyl acrylate.
Preferably, the method further comprises:

providing a reaction injection device (40), which comprises at least two storage tanks (48, 49) for accommodating the components of the polyurethane composition, a vacuumizing device (50) and feeding units (44a, 44b), wherein each of the feeding units (44a, 44b) is connected to the storage tank (48, 49) via a feeding line (41, 42) and a mixing unit (43), the components from the feeding units (44a, 44b) are mixed together;
wherein the rim of the mold is sealed and the mold is connected to at least an injection line (31), which can be used for vacuumizing the mold (55) and supplying the mixed components to the mold (55); and the mold comprises optionally a drying channel (32) for providing a drying gas (33); during the vacuum infusion, the drying gas is supplied to the mold to dry the core material, the reinforcing material and/or the flow medium (21) placed in the mold; and the mold (55) is vacuumized by means of a vacuum source (34), and the mold is connected to the reaction injection device (40) via the injection line (31) and the injection line (45); and the mold can be vacuumized via the injection line (45) through a laterally closable outlet (46), which is connected to a vacuum source (47);
drying the mold (55) and the reinforcing material, core material and/or flow medium (21) contained therein, the injection line (45), and the optional feeding unit (44a, 44b)/mixing unit (43), wherein optionally the drying gas (33) can be introduced via the drying channel (32);
beginning the vacuum infusion process with introducing the degassed components in the feeding lines (41, 42) from the storage tanks (48, 49) into the feeding units (44a, 44b) of the reaction injection device (40), and obtaining the polyurethane composition from the components in the mixing unit (43), wherein the outlet (46) of the vacuum source (47) is closed before the polyurethane composition arrives;
injecting the polyurethane composition into the mold (55) via the injection line (31) while vacuumizing the mold (55) via the drying channel (32) by the vacuum source (34), wherein the injection pressure measured at the injection port of the injection line (31) is kept lower than the external atmospheric pressure.

The flow medium of the present invention refers to a substance having a porous structure, which may be a material obtained by braiding, weaving, knitting, extruding or crocheting, a foam or a substance having a sieve or a network structure itself. Specifically, it includes but is not limited to woven flow meshes, pressed flow meshes, continuous fiber felts and hybrid flow meshes, for example, those obtained by mixing two or more of fiber fabrics such as woven flow meshes, pressed flow meshes, continuous fiber felts and chopped fiber felts. Those skilled in the art are familiar

with materials that can be used as a flow medium, including but not limited to, polystyrene (PS), polyurethane (PUR), polyphenylene oxide (PPO), polypropylene, ABS, and glass fiber fabrics. Porous components or flow media are primarily used to aid in vacuumizing during the drying process and in guiding flow during the introduction of the polyurethane composition/liquid material.

The method of the present invention may further comprise a peel ply. The peel ply which can be used in the present invention, preferably polyester peel ply, refers to a peel ply made from polyester fiber. Polyester fiber (PET fiber) or PET fiber for short, commonly referred to as " dacron", is a general term for fibers made from polyesters obtained by polycondensation of various diols and aromatic dicarboxylic acids or esters thereof. Preferably, the polyester peel ply is selected from the group consisting of plain weaves, twill weaves, satin weaves made of continuous fibers by weaving methods or fabrics made by knitting methods or fabrics directly made by stitching methods.

The polyester peel ply may be placed between the reinforcing material and the mold, or may be placed between the reinforcing material and/or the core material and the flow medium such as a flow mesh.

Molds that can be used in the present invention include, but are not limited to, molds of wind turbine blades and/or components thereof, molds of aircrafts and/or components thereof, molds of hulls and/or component thereof, molds of vehicle bodies and/or components thereof, and the like. In an example of the present invention, the mold is preferably a mold that can be used to produce wind turbine blades and/or components thereof in a polyurethane vacuum infusion process. The molds may have a heating function.

Optionally, the method for heating the peel ply, the reinforcing material, the flow medium and/or the core material of the present invention is one, two or more selected from the group consisting of mold heating, electric blanket heating, electric film heating, microwave heating, infrared heating and hot air heating. In the electric blanket heating and the electric film heating, the electric blanket and the electric film are placed under the mold or cover the film outside, and heat by supplying electric current. Other conventional heating methods in the art can all be used in the present invention.

The experimental results show that the present invention provides a method for preparing a polyurethane composite, which has higher efficiency and higher accuracy and is more energy-saving. Said method can show intuitively the time for infusing the polyurethane composition after completing the dehumidification, thereby greatly improving the production efficiency of polyurethane composites, saving costs and being more conducive to environmental protection. In particular, for large polyurethane products, the method of the present invention reduces the adverse effects due to the harsh application conditions of polyurethanes (e.g., sensitivity to water) effectively, and achieves high production efficiency economically and efficiently. In addition, the polyurethane composition of the present invention has a longer pot life, so that polyurethane composites having uniform quality and excellent physical properties can be obtained when preparing large polyurethane products. The polyurethane vacuum infusion process for large parts usually requires covering the film/bag film to maintain the vacuum degree. But the film is prone to leakage and breakage, resulting in an increase in internal humidity, which is detrimental to local or overall dehumidification. The use of humidity indicating agents/papers can help to find the leakage and breakage of the film quickly and accurately, so that it can be repaired in time to avoid or reduce its adverse effects.

The polyisocyanate of the present invention may be an organic polyisocyanate which may be any aliphatic, cycloaliphatic or aromatic isocyanate known for preparing polyurethane composites. Examples thereof include, but are not limited to, toluene diisocyanate (TDI), diphenylmethane diisocyanate (MDI), polyphenylpolymethylene polyisocyanate (pMDI), 1,5-naphthalene diisocyanate (NDI), hexamethylene diisocyanate (HDI), methylcyclohexyl diisocyanate (TDI), 4,4'-dicyclohexylmethane diisocyanate, isophorone diisocyanate (IPDI), p-phenylene diisocyanate (PPDI), p-xylene diisocyanate (XDI), tetramethylxylene diisocyanate (TMXDI), and polymers or combinations thereof. The isocyanate useful in the present invention has a functionality of preferably 2.0 to 3.5, particularly preferably 2.1 to 2.9. The isocyanate has a viscosity of preferably 5 to 700 mPa·s, particularly preferably 10 to 300 mPa·s, measured at 25 °C according to DIN 53019-1-3.

When used in the present invention, the organic polyisocyanate includes isocyanate dimer, trimer, tetramer, pentamer or combinations thereof.

In a preferred example of the present invention, the isocyanate component A) is selected from the group consisting of diphenylmethane diisocyanate (MDI), polyphenylpolymethylene polyisocyanate (pMDI), and polymers, prepolymers or combinations thereof.

Blocked isocyanates can also be used as the isocyanate component A), which can be prepared by reacting an excess of organic polyisocyanate or a mixture thereof with a polyol compound. These compounds and their preparation methods are well known to those skilled in the art.

The polyurethane reaction system of the present invention comprises one or more organic polyols b1). The organic polyol is present in a content of 21 to 60 wt%, based on the total weight of the polyurethane reaction system as 100 wt%. The organic polyol may be an organic polyol commonly used in the art for preparing polyurethanes, including but not limited to polyether polyols, polyether carbonate polyols, polyester polyols, polycarbonate diols, polymer polyols, vegetable oil based polyol or a combination thereof.

The polyether polyol can be prepared by a known process, for example, by reacting an olefin oxide with a

starter in the presence of a catalyst. The catalyst is preferably, but not limited to, a basic hydroxide, a basic alkoxide, antimony pentachloride, boron fluoride etherate, or a mixture thereof. The olefin oxide is preferably but not limited to tetrahydrofuran, ethylene oxide, propylene oxide, 1,2-butylene oxide, 2,3-butylene oxide, styrene oxide, or a mixture thereof, particularly preferably ehylene oxide and/or propylene oxide. The starter is preferably but not limited to a polyhydroxy compound or a polyamine compound. Said polyhydroxy compound is preferably but not limited to water, ethylene glycol, 1,2-propanediol, 1,3-propanediol, diethylene glycol, trimethylolpropane, glycerol, bisphenol A, bisphenol S or a mixture thereof. Said polyamine compound is preferably but not limited to ethylene diamine, propylene diamine, butanediamine, hexanediamine, diethylenetriamine, toluenediamine or a mixture thereof.

Methods for measuring the hydroxyl value are well known to those skilled in the art and are disclosed, for example, in Houben Weyl, Methoden der Organischen Chemie, vol. XIV/2 Makromolekulare Stoffe, p. 17, Georg Thieme Verlag; Stuttgart 1963. The entire contents of this document are incorporated herein by reference.

As used herein, unless otherwise indicated, the functionality and hydroxyl value of organic polyols refer to average functionality and average hydroxyl value.

Optionally, the polyurethane reaction system of the present invention further comprises one or more compounds b2) having the structure of formula (I)

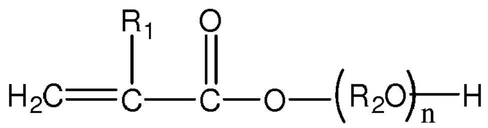

I ,

wherein $R_1$ is selected from hydrogen, methyl or ethyl; $R_2$ is selected from alkylene groups having 2 to 6 carbon atoms; and n is an integer selected from 1 to 6.

In a preferred example of the present invention, $R_2$ is selected from the group consisting of ethylene, propylene, butylene, pentylene, 1-methyl-1,2-ethylene, 2-methyl-1,2-ethylene, 1-ethyl-1,2-ethylene, 2-ethyl-1,2-ethylene, 1-methyl-1,3-propylene, 2-methyl-1,3-propylene, 3-methyl-1,3-propylene, 1-ethyl-1,3-propylene, 2-ethyl-1,3-propylene, 3-ethyl-1,3-propylene, 1-methyl-1,4-butylene, 2-methyl-1,4-butylene, 3-methyl-1,4-butylene and 4-methyl-1,4-butylene, 2,2-di(4-phenylene)-propane, 1,4-xylylene, 1,3-xylylene, 1,2-xylylene.

In a preferred example of the present invention, the component b2) is selected from the group consisting of hydroxyethyl methacrylate, hydroxypropyl methacrylate, hydroxybutyl methacrylate, hydroxyethyl acrylate, hydroxypropyl acrylate, hydroxybutyl acrylate or a combination thereof.

The compound of the formula (I) can be prepared by a method generally used in the art, for example, by esterification reaction of (meth)acrylic anhydride or (meth)acrylic acid, (meth)acryloyl halide compound with $HO-(R_2O)_n-H$. The preparation method is well known to those skilled in the art, for example, in "Handbook of Polyurethane Raw Materials and Auxiliaries" (Yijun Liu, published on April 1, 2005), Chapter 3; "Polyurethane Elastomer" (Houjun Liu, published in August 2012), Chapter 2. The entire contents of these documents are incorporated herein by reference.

The polyurethane reaction system of the present invention further comprises C) a free radical initiator. The radical initiator used in the present invention may be added to the polyol component or the isocyanate component or both components. Useful free radical initiators include, but are not limited to, peroxides, persulfides, peroxycarbonates, peroxyboric acid, azo compounds, or other suitable free radical initiators which can initiate the curing of double bond containing compounds, examples of which include tert-butyl peroxy isopropyl carbonate, tert-butyl peroxy-3,5,5-trimethylhexanoate, methyl ethyl ketone peroxide, and cumene hydroperoxide. The radical initiator is usually present in a content of 0.1 to 8 wt%, based on the total weight of the polyurethane reaction system of the present invention as 100 wt%. In addition, an accelerator such as a cobalt compound or an amine compound may also be present.

Optionally, the polyurethane reaction system may further comprise a catalyst for catalyzing the reaction of isocyanate groups (NCO) with hydroxyl groups (OH). Suitable polyurethane reaction catalysts are preferably, but not limited to, amine catalysts, organometallic catalysts, or mixtures thereof. The amine catalyst is preferably but not limited to triethylamine, tributylamine, triethylenediamine, N-ethylmorpholine, N,N,N',N'-tetramethyl-ethylenediamine, pentamethyldiethylene-triamine, N-methylaniline, N,N-dimethylaniline, or a mixture thereof. The organometallic catalyst is preferably but not limited to an organotin compound such as tin (II) acetate, tin (II) octoate, tin ethylhexanoate, tin laurate, dibutyl tin oxide, dibutyltin dichloride, dibutyltin diacetate, dibutyltin maleate, dioctyltin diacetate, or a mixture thereof. The catalyst is used in an amount of 0.001 to 10 wt%, based on the total weight of the polyurethane reaction system of the present invention as 100 wt%.

In an example of the present invention, in the polyaddition reaction of isocyanate groups and hydroxyl groups, the isocyanate groups may be those contained in the organic polyisocyanate (component A) or may also be those

contained in the reaction intermediate of the organic polyisocyanate (component A) with the organic polyol (component b1)) or component b2). The hydroxyl groups may be those contained in the organic polyol (component b1)) or component b2) or may also be those contained in the reaction intermediate of the organic polyisocyanate (component A) with the organic polyol (component b1) or component b2)).

In an example of the present invention, the radical polymerization reaction is a polyaddition reaction of ethylenic bonds, wherein the ethylenic bonds may be those contained in the component b2) or may also be those contained in the reaction intermediate of the component b2) with the organic polyisocyanate.

In the examples of the present invention, the polyurethane polyaddition reaction (i.e., the polyaddition reaction of isocyanate groups with hydroxyl groups) is present simultaneously with a radical polymerization reaction. It is well known to those skilled in the art that suitable reaction conditions can be selected such that the polyurethane polyaddition reaction and the radical polymerization reaction are carried out in succession. However, the polyurethane matrix thus obtained has a different structure from that of a polyurethane matrix obtained by simultaneous polyaddition reaction and radical polymerization reaction. Thus, the mechanical properties and processability of the prepared polyurethane composites are different.

Optionally, the above polyurethane reaction system may further comprise an auxiliary or additive, including but not limited to a filler, an internal demolding agent, a flame retardant, a smoke suppressant, a dye, a pigment, an antistatic agent, an antioxidant, a UV stabilizer, a diluent, a defoaming agent, a coupling agent, a surface wetting agent, a leveling agent, a water scavenger, a catalyst, a molecular sieve, a thixotropic agent, a plasticizer, a foaming agent, a foam stabilizer, a foam homogenizing agent, an inhibitor against free radical reaction or a combination thereof. These components may optionally be included in the isocyanate component A) and/or the polyurethane reaction system B) of the present invention. These components may also be stored separately as a component D), which is mixed with the isocyanate component A) and/or the polyurethane reaction system B) of the present invention and then used for the preparation of polyurethane composites. The selection of the above-mentioned auxiliaries or additives and the above-mentioned content that is not described in detail can be found in CN104974502A, which is entirely incorporated herein by reference.

A second aspect of the present invention is to provide a polyurethane composite which is obtained by the method of the present invention for preparing a polyurethane composite by a vacuum infusion process.

A third aspect of the present invention is to provide use of the polyurethane composite of the present invention in a wind turbine blade.

A fourth aspect of the present invention is to provide a polyurethane product comprising a polyurethane composite obtained by the method for preparing a polyurethane composite by a vacuum infusion process.

Preferably, the polyurethane product is selected from the group consisting of a wind turbine blade, a radome, a ship part, a rail, a single or sandwich sheet, preferably a spar cap, a web plate, a blade root and/or a blade housing of a wind turbine blade.

The method of the present invention for detecting the degree of dehumidification in a polyurethane vacuum infusion process, the related computer device and storage medium are described below with reference to description of block diagrams, block diagrams and/or flow diagrams of the present invention. The description of block diagrams and/or each block of the block diagrams, as well as the description of flow diagrams and/or combinations of block diagrams can be implemented by instructions of the computer program . These computer program instructions may be provided to a processor of a general computer, a special computer or other programmable data processing devices to form a machine, so that the instructions executed by the processor of the computer or other programmable data processing devices create parts for implementing functions/operations specified in these flow diagrams and/or blocks and/or one or more flow block diagrams.

The computer program instructions may be stored in a computer readable memory and instruct a computer or other programmable processors to perform functions in a particular manner so that the instructions stored in the computer readable memory constitute products comprising instruction parts for implementing functions/operations specified in flow diagrams and/or one or more blocks in the block diagrams.

These computer program instructions can be loaded onto a computer or other programmable data processors so that a series of operation steps may be executed on the computer or other programmable processors. In this way, it constitutes a computer-implemented process so that the instructions executed on the computer or other programmable data processors provide steps for implementing functions/operations specified in the flow diagrams and/or one or more block in the block diagrams. It should also be noted that in some alternative embodiments, the functions/operations shown in the blocks may occur in an order different from that shown in the flow diagrams. For example, two blocks shown in succession may be executed substantially concurrently or these blocks may be executed in a reverse order, depending on the function/operation involved.

Figure 4 is a schematic flow diagram S100 of an embodiment of the method of the present invention for detecting the degree of dehumidification in a polyurethane vacuum infusion process.

As shown in S100 in Figure 4, the method includes the steps of: comparing the color information of the humidity/moisture indicating paper/agent to be detected with the target color information, until the color information of the humidity/moisture indicating paper/agent to be detected conforms to the target color information (step SI), wherein each of the target color information corresponds to the dehumidification requirement fulfilled in the polyurethane vacuum infusion process of the present invention, in particular, the method for preparing a polyurethane composite by a vacuum infusion process which has been determined by experiments (for example, the foregoing Examples 1 to 11).

Preferably, the above step S1 can be implemented in the computer device of the present invention.

Preferably, as shown in Figure 4, the method S100 may further include the steps of: obtaining a moisture/humidity detection result based on the comparison of the color information of the humidity/moisture indicating paper/agent to be detected with the target color information (step S2).

Furthermore, the color information of the humidity/moisture indicating paper/agent to be detected may preferably be added to the database of target color information, so that the database may be further improved.

A computer device 100 of an embodiment of the present invention is shown in Figure 5. The computer device 100 comprises a database unit 101, which stores a database comprising multiple pieces of target color information of humidity/moisture indicating paper/agents (e.g., color values as set forth in Examples 1 to 11 of the present invention) corresponding to the dehumidification requirements fulfilled in the polyurethane vacuum infusion process of the present invention, in particular, the method for preparing a polyurethane composite by a vacuum infusion process which have been determined by experiments.

The device 100 may further comprise a color comparing unit 102, which is configured to compare the color information of the humidity/moisture indicating paper/agent to be detected with the target color information, so that it can be determined whether the color information of the humidity/moisture indicating paper/agent conforms to the target color information, in particular, the target color information of the humidity/moisture indicating paper/agent corresponding to the dehumidification requirements in the method for preparing a polyurethane composite by a vacuum infusion process (for example, the color values as set forth in Examples 1 to 11 of the present invention).

The device 100 may further comprises an output unit for detection results 103, which is configured to output a detection result based on the result of the comparison.

Although the present invention has been described as above with embodiments of the method for detecting the degree of dehumidification in a polyurethane vacuum infusion process and the computer device, the present invention is not limited to these embodiments. The present invention may also be embodied in a form of a computer program implementing the functions of the computer device described above or a storage medium having the computer program stored thereon.

Figure 6 shows a schematic block diagram of a computer device 200 according to another embodiment of the present invention. As shown in Figure 6, the computer device 200 comprises a memory 201, a processor 202 and a computer program stored on the memory 201 and operable on the processor 202, wherein the processor executes the computer program to implement the steps of the method S100 for detecting the degree of dehumidification in a vacuum infusion process according to an embodiment of the present invention.

The memory 201 comprises a database comprising multiple pieces of target color information of humidity/moisture indicating paper/agents corresponding to the dehumidification requirements fulfilled in the polyurethane vacuum infusion process of the present invention, in particular, the method for preparing a polyurethane composite by a vacuum infusion process which have been determined by experiments.

Furthermore, as described above, the method of the present invention may also be embodied as a storage medium having a program stored thereon, wherein the program is executed by a processor to implement the method S100 for detecting the degree of dehumidification in a polyurethane vacuum infusion process according to an embodiment of the present invention.

The storage medium of the present invention may be any type of storage media, such as a disk (for example a magnetic disk, an optical disk, etc.), a card (for example a memory card, an optical card, etc.), a semiconductor memory (for example a ROM, a nonvolatile memory, etc.), and a tape (for example a magnetic tape, cassette tapes, etc.).

By storing in these storage media a computer program for rendering a computer to execute the method for detecting the degree of dehumidification in a polyurethane vacuum infusion process in the above embodiments or a computer program for rendering the computer to realize the functions of the computer device in the above embodiments and by circulating these computer programs, the costs can be reduced and the portability and versatility can be improved.

In addition, the storage medium is inserted in a computer, and the computer program stored in the storage medium is read by a computer and stored in the memory. The processor (CPU: Central Processing Unit, MPU: Micro Processing Unit) of the computer reads and executes the computer program from the memory. In this way, the method for detecting the degree of dehumidification in a polyurethane vacuum infusion process in the above embodiment can be executed and the functions of computer devices in the above embodiment can be realized.

As mentioned above, we have unexpectedly found that the method of the present invention for detecting the degree of dehumidification in a polyurethane vacuum infusion process makes it possible to detect the degree of dehumidification in the polyurethane vacuum infusion process objectively and accurately, improve production efficiency, save

costs, and be more economical and environmentally friendly.

It is to be understood by those skilled in the art that the present invention is not limited to the embodiments described above, and the invention may be embodied in many other forms without departing from the spirit and scope of the invention. The present examples and embodiments are to be considered as illustrative and not restrictive, and the invention may cover various modifications and replacements, without departing from the spirit and scope of the invention as defined by the appended claims.

Unless defined otherwise, all technical and scientific terms used herein have the same meaning as they are generally understood by those skilled in the art of the present invention. When definitions of the terms in this specification conflicts with the meaning generally understood by those skilled in the art of the present invention, the definitions described herein shall apply.

Unless stated otherwise, all values expressing quantities of ingredients, reaction conditions, and the like, as used herein, are understood to be modified by the term "about."

As used herein, "and/or" refers to one or all of the elements mentioned.

"Including" and "comprising", as used herein, cover the cases where only the mentioned elements are present, as well as the cases where there are other unmentioned elements in addition to the mentioned elements.

Unless indicated otherwise, all percentages herein are percentages by weight.

The invention is now described using the examples by way of illustration without limitation.

**Examples**

Description of tested performance parameters in the examples of the present application: Functionality refers to a value determined according to the formula in the industry:

$$\text{Functionality} = \text{hydroxyl value} * \text{molecular weight} / 56100;$$

wherein the molecular weight is determined by GPC high performance liquid chromatography;

Isocyanate index refers to a value determined by the following formula:

$$\text{Isocyanate index (\%)} = \frac{\text{Moles of isocyanate groups (NCO groups) in component A}}{\text{Moles of groups reactive toward isocyanate groups in component B}} \text{X } 100\%$$

NCO content refers to the content of NCO groups in the system, measured according to GB/T 12009.4-2016.

Table 1 - Description of raw materials:

| Name of raw materials | Specification / type | Suppliers |
|---|---|---|
| Polyol | Baydur 78BD085 | Covestro Polymers (China) Co., Ltd. |
| Isocyanate | Desmodur 44CP20 | Covestro Polymers (China) Co., Ltd. |
| Biaxial fiber glass fabric | EKT811 (+45°/-45°) Specification: 808g/$m^2$ | Chongqing Polycomp International Corp. |
| Injection hose / Omega hose | Material: PE | Shanghai Leadgo-tech Co., Ltd |
| Flow mesh | Material: PE | Shanghai Leadgo-tech Co., Ltd |
| PVC foam core material | Specification: 60kg/$m^3$ | 3A Composites (China) Ltd |
| Polyester peel ply | Gram weight: 95g/$m^2$ | Shanghai Leadgo-tech Co., Ltd |
| Film/vacuum bag film | Thickness: 50 μm | Shanghai Leadgo-tech Co., Ltd |

(continued)

| Name of raw materials | Specification / type | Suppliers |
|---|---|---|
| adhesive strip | Type: WD209 | Shanghai KangDa New Materials Co., Ltd |
| Warming blanket | Specification: width of 1m, length of 2m, thickness of 30 mm | Related market |
| Cobalt chloride paper | Specification: 5 stacks per box, 100 pieces, outer packaging size 73*30*15mm, about 20g | Shanghai Sss Reagent Co., Ltd |
| colorimeter | spectro-guide 45/0 gloss | BYK |

**Description of test methods:**

Temperature test: an infrared thermometer is used to monitor the surface temperature; Color/Color Value Test: the color value test of the present invention refers to a test in which the color (for example, the color of the paper before and after the color change) is tested using a colorimeter (for example spectro-guide 45/0 gloss provided by BYK) according to CIE 1976 L*a*b* standard, so that L*a*b* color values are obtained.

**Examples:**

Examples 1 to 11 and Comparative Examples 1 to 4:

Two layers of biaxial glass fiber fabrics with length × width of 500*500 mm were laid on the mold. Then, a PVC foam core material with length × width of 400*400 mm was placed on the glass fiber fabrics, wherein the grooved side pointed upward. Afterwards, two more layers of biaxial fiber glass fabrics with length × width of 500*500 mm were laid on the foam core material. A piece of peel ply was placed on the second layer of glass fiber fabric. The flow mesh was placed on the peel ply. An injection hose with a length of 300 mm was obtained by cutting and placed next to the flow mesh. A few pieces of cobalt chloride paper were laid on the peel ply. Two loops of adhesive sealing strips were stuck around each layer laid in the mold. Then, said layers were sealed with two layers of vacuum bag film.

Firstly, the heating temperature of the mold was set to 45 °C, which is then gradually lowered to 35 °C (gradually changed from high to low temperature with the heating time). The system was heated and the vacuum pump was connected to the injection hose. The system was vacuumized to 0 to 20 mbar and covered and heated by a warming blanket (Comparative Example 1: the color was unchanged and still pink; Example 1 to 11: until the color of the cobalt chloride paper was changed to blue; the values of the colorimeter are shown in Tables 2 and 3). At this time, the polyurethane composition could be infused. After completing the infusion, it was cured by heating and then demolded after curing. The auxiliary materials such as the peel ply and the flow mesh were removed. The obtained polyurethane composite was inspected and found to have no surface defects (as shown in the left picture in Figure 2) and meet the requirements in terms of all physical properties.

According to the above steps, the layers were laid and dehumidified by heating. When the color did not change (Comparative Example 1: pink), the polyurethane composition was infused. The obtained polyurethane composite was inspected, and the surface thereof was found to have defects (as shown in the right picture in Figure 2). Show), indicating that dehumidification did not reach the expected degree.

Table 2 - Color values of Comparative Examples 1 to 4 and Example 1

| Color values | Comparative Example 1 | Comparative Example 2 | Comparative Example 3 | Comparative Example 4 | Comparative Example 1 |
|---|---|---|---|---|---|
| L* | 84.83 | 85.25 | 75.74 | 74.74 | 69.58 |
| a* | 6.65 | 4.14 | -8.14 | -8.06 | -11.11 |
| b* | 0.41 | 1.64 | -6.77 | -10.85 | -18.5 |

Table 3 - Color values of Examples 2 to 11

| Color values | 2 | 3 | 4 | 5 | 6 | 7 | 8 | 9 | 10 | 11 | Average value |
|---|---|---|---|---|---|---|---|---|---|---|---|
| L* | 70.29 | 70.32 | 69.77 | 71.59 | 69.08 | 74.43 | 73.73 | 72.96 | 75.12 | 72.75 | 72 |
| a* | -10.71 | -10.03 | -9.5 | -10.31 | -10.33 | -10.03 | -9.73 | -9.21 | -9.76 | -9.84 | -9.95 |
| b* | -19.49 | -21.3 | -22.75 | -19.87 | -20.09 | -17.34 | -18.7 | -20.16 | -17.55 | -18.06 | -19.5 |

After repeated tests, we unexpectedly found that the polyurethane composition could be infused when the humidity indicating agent/paper changed its color, preferably when the color changed to a color having a certain color value displayed by colorimeter, i.e. when the desired degree of dehumidification was reached.

The above experimental results show that the method according to embodiments of the present invention can indicate accurately the time when the dehumidification is completed and the polyurethane composition can be infused, thereby characterizing the dehumidification degree objectively and accurately and improving the production efficiency greatly. Moreover, it can indicate the damage and leakage of the film/bag film accurately, thereby avoiding delays and losses.

On the contrary, the degree of dehumidification was insufficient when the color of the cobalt chloride paper was pink (i.e., the color did not change). For example, in the case of Comparative Example 1, the polyurethane composition was infused and the surface condition of the obtained polyurethane composite is shown in the right picture in Figure 2. It can be seen that there were bubbles on the surface and the surface condition was not good.

## Claims

1. A method for preparing a polyurethane composite by a vacuum infusion process, comprising:

   a) placing at least a reinforcing material, a core material and/or a flow medium on a mold, and placing at least a humidity/moisture indicating paper/agent on the reinforcing material, the core material or the flow medium;
   b) dehumidifying under vacuum until the humidity/moisture indicating paper/agent changes color;
   c) introducing a polyurethane composition, and demolding after curing to obtain the polyurethane composite.

2. The method of claim 1, **characterized in that** the humidity/moisture indicating paper/agent is selected from the group consisting of cobalt chloride, and the color is changed to blue.

3. The method according to claim 2, **characterized in that** the color is changed to any color selected from the group consisting of those with range b* < -15, preferably < -17, more preferably -25 to -18 in color values (according to test method CIE 1976 L*a*b*).

4. The method according to claim 3, **characterized in that** the color is changed to any color selected from the group consisting of those with range L* ≤ 80, preferably ≤ 75, more preferably ≤ 73; a* < -5, preferably < -7, more preferably < -8 in color values (according to test method CIE 1976 L*a*b*).

5. The method according to any one of claims 1 to 4, **characterized in that** the dehumidification comprises heating, wherein the heating temperature is firstly set to 40 to 50 °C, preferably 40 to 45 °C, and then gradually reduce to 20 to 38 °C.

6. The method according to any one of claims 1 to 4, wherein the step b) further comprises:

   covering said at least a reinforcing material, a core material and/or a flow medium with a first film, and sealing the rim of the first film with the mold, and vacuuming between the first film and the mold;
   laying a second film to cover the first film, fixing the second film, sealing the rims of the first film and the second film, wherein an air inlet channel and an air outlet channel are reserved;
   heating the mold, while filling hot air between the first film and the second film to provide a temperature close to the mold temperature for the upper surface of the first film.

7. The method according to any one of claims 1 to 4, **characterized in that**
   the reinforcing material is selected from the group consisting of entangled glass fiber layers, glass fiber woven fabrics

and glass fiber gauzes, cut or ground glass fibers or mineral fibers, as well as fiber mats, fiber nonwovens and fiber knits based on polymer fibers, mineral fibers, carbon fibers, glass fibers or aramid fibers and mixtures thereof, more preferably glass fiber mats, glass fiber nonwovens, glass fiber woven fabrics and mixtures thereof, and **characterized in that**
the core material is selected from the group consisting of balsa wood, PVC foam, SAN foam, polyurethane foam, PS foam, PMI foam and PET foam,
and **characterized in that** the flow medium comprises a peel ply, and said peel ply is preferably a polyester peel ply.

**8.** The method according to any one of claims 1 to 4, **characterized in that** the polyurethane composition comprises the following components:

a component A, comprising one or more organic polyisocyanates;
a component B, comprising:

b1) one or more organic polyols, which is present in a content of 21 to 60 wt%, based on the total weight of the polyurethane composition as 100 wt%;
b2) one or more compounds having the structure of formula (I)

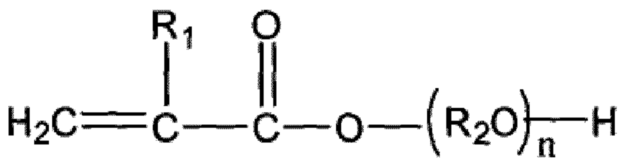

I

wherein R1 is selected from hydrogen, methyl or ethyl; R2 is selected from alkylene groups having 2 to 6 carbon atoms, 2,2-di(4-phenylene)-propane, 1,4-xylylene, 1,3-xylylene, 1,2-xylylene; n is an integer selected from 1 to 6; and

a component C, a free radical initiator.

**9.** The method according to claim 8 or 9, **characterized in that** the component b2) is present in a content of 4.6 to 33 wt% based on the total weight of the polyurethane composition as 100 wt%, and **characterized in that** the component b2) is one, two or more selected from the group consisting of hydroxyethyl methacrylate, hydroxypropyl methacrylate, hydroxybutyl methacrylate, hydroxyethyl acrylate, hydroxypropyl acrylate and hydroxybutyl acrylate.

**10.** The method according to any one of claims 1 to 4, **characterized in that** the method further comprises:

providing a reaction injection device (40), which comprises at least two storage tanks (48, 49) for accommodating the components of the polyurethane composition, a vacuumizing device (50) and feeding units (44a, 44b), wherein each of the feeding units (44a, 44b) is connected to the storage tank (48, 49) via a feeding line (41, 42) and a mixing unit (43), the components from the feeding units (44a, 44b) are mixed together;
wherein the rim of the mold is sealed and the mold is connected to at least an injection line (31), which can be used for vacuumizing the mold (55) and supplying the mixed components to the mold (55); and the mold comprises optionally a drying channel (32) for providing a drying gas (33); during the vacuum infusion, the drying gas is supplied to the mold to dry the core material, the reinforcing material and/or the flow medium (21) placed in the mold; and the mold (55) is vacuumized by means of a vacuum source (34), and the mold is connected to the reaction injection device (40) via the injection line (31) and the injection line (45); and the mold can be vacuumized via the injection line (45) through a laterally closable outlet (46), which is connected to a vacuum source (47);
drying the mold (55) and the reinforcing material, core material and/or flow medium (21) contained therein, the injection line (45), and the optional feeding unit (44a, 44b)/mixing unit (43), wherein optionally the drying gas (33) can be introduced via the drying channel (32); beginning the vacuum infusion process with introducing the degassed components in the feeding line (41, 42) from the storage tanks (48, 49) into the feeding units (44a, 44b) of the reaction injection device (40), and obtaining the polyurethane composition from the components in

the mixing unit (43), wherein the outlet (46) of the vacuum source (47) is closed before the polyurethane composition arrives;
injecting the polyurethane composition into the mold (55) via the injection line (31) while vacuumizing the mold (55) via the drying channel (32) by the vacuum source (34), wherein the injection pressure measured at the injection port of the injection line (31) is kept lower than the external atmospheric pressure.
A polyurethane composite obtained by the method for preparing a polyurethane composite by a vacuum infusion process according to any one of claims 1 to 10.

**11.** Use of a polyurethane composite obtained by the method for preparing a polyurethane composite by a vacuum infusion process according to any one of claims 1 to 15 in a wind turbine blade, a radome, a ship part, a rail, a single or sandwich sheet, preferably a spar cap, a web plate, a blade root and/or a blade housing of a wind turbine blade.

**12.** A method for detecting the degree of dehumidification in a polyurethane vacuum infusion process, comprising:

obtaining the color information of a humidity/moisture indicating paper/agent to be detected;
comparing the color information of the humidity/moisture indicating paper/agent to be detected with the target color information, until the color information of the humidity/moisture indicating paper/agent to be detected conforms to the target color information.

**13.** The detection method according to claim 12, **characterized in that** it further comprises:

storing the target color information beforehand;
wherein the color information is measured by a color detecting instrument.

**14.** A computer device comprising:

a database unit, which stores multiple pieces of target color information of detected humidity/moisture indicating paper/agents that achieve the dehumidification standard of a polyurethane vacuum infusion process;
a color comparing unit, which is configured to compare the color information of the humidity/moisture indicating paper/agent to be detected with the target color information, so that it can be determined whether the color information of the humidity/moisture indicating paper/agent conforms to the target color information;
an output unit for detection results, which is configured to output a detection result based on the result of the comparison.

**15.** A storage medium having a computer program stored thereon, wherein the computer program is executed by a processor to implement the detection method according to claim 12 or 13.

Fig. 1

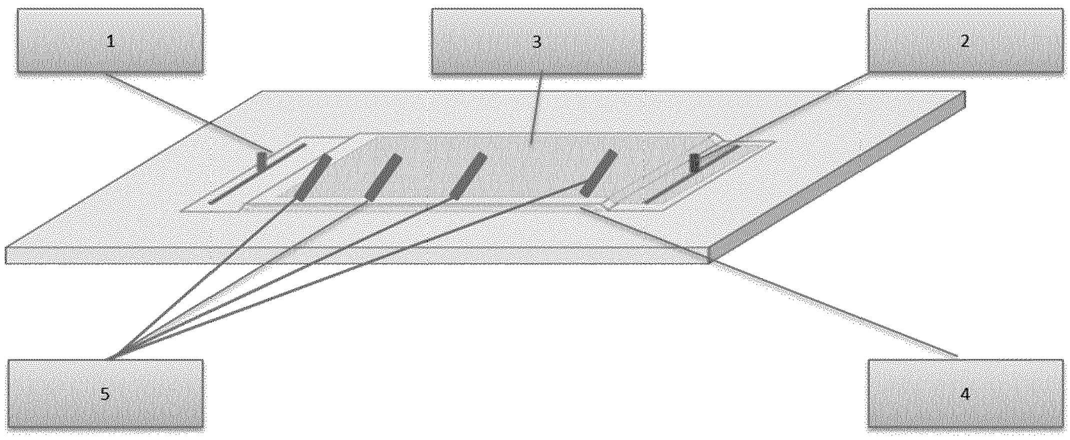

Fig. 2

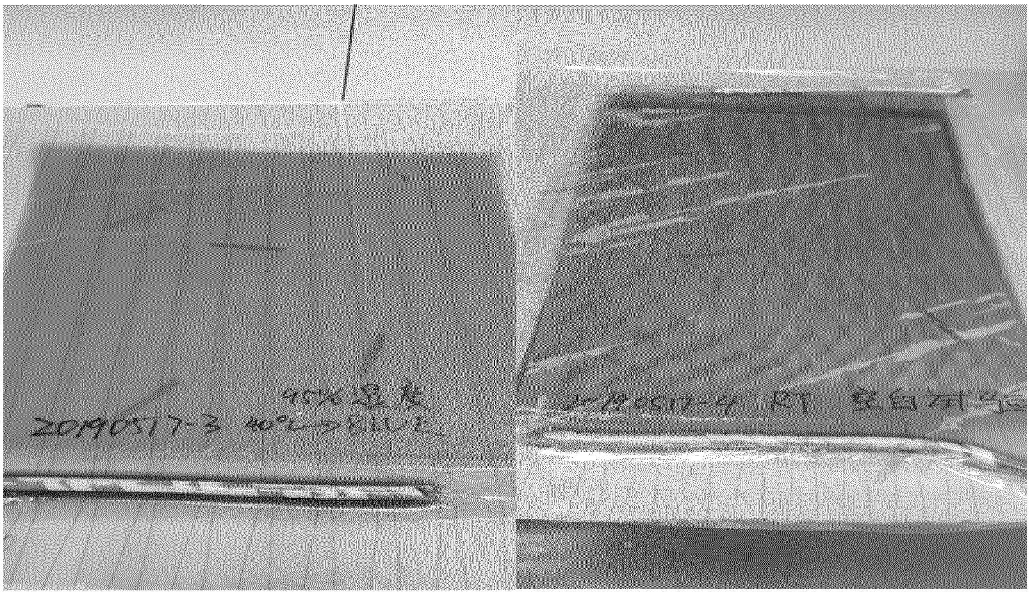


20190517-3 95% Humidity 40 °C → Blue

20190517-4 RT Blank test

Fig. 3

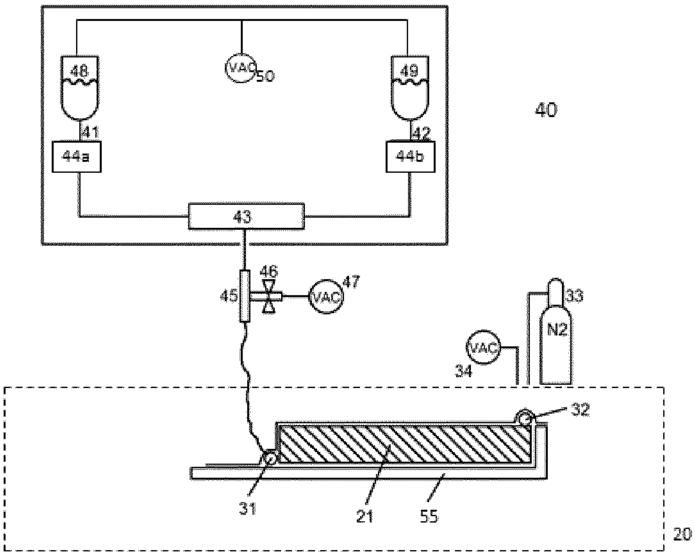
48
VAC
50
49
40
41
42
44a
44b
43
46
45
VAC
47
33
N2
VAC
34
32
31
21
55
20

Fig. 4

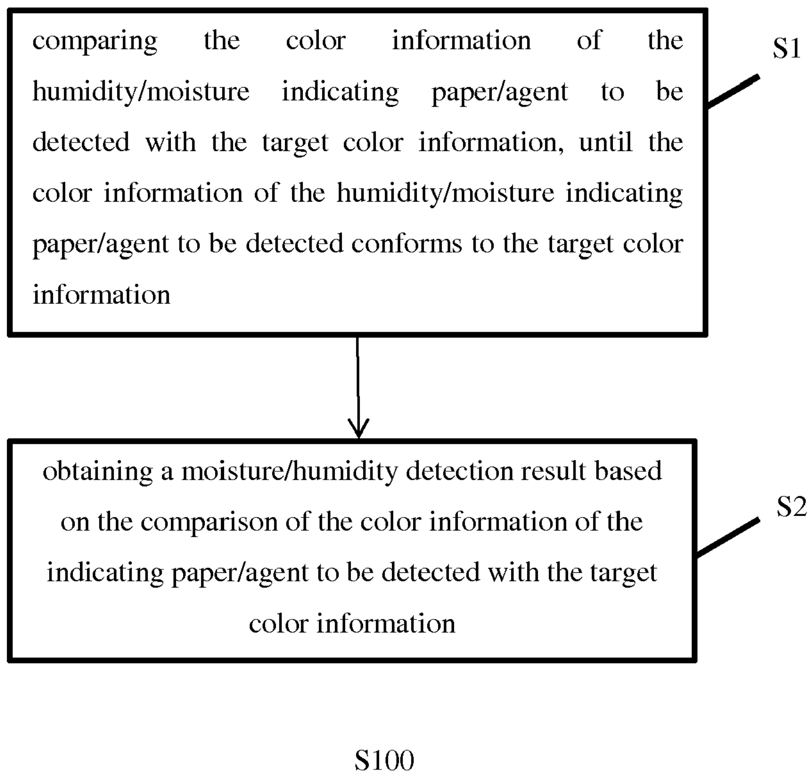
comparing the color information of the humidity/moisture indicating paper/agent to be detected with the target color information, until the color information of the humidity/moisture indicating paper/agent to be detected conforms to the target color information
S1
obtaining a moisture/humidity detection result based on the comparison of the color information of the indicating paper/agent to be detected with the target color information
S2
S100

Fig. 5

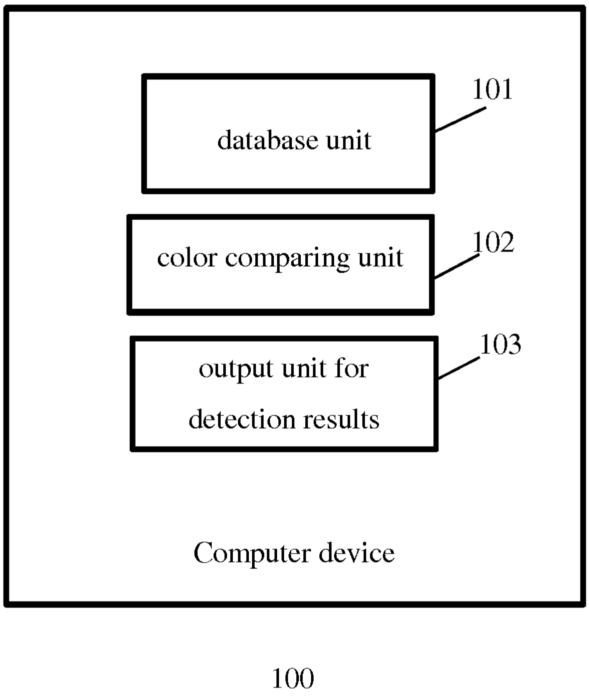
database unit
101
color comparing unit
102
output unit for
detection results
103
Computer device
100

Fig. 6

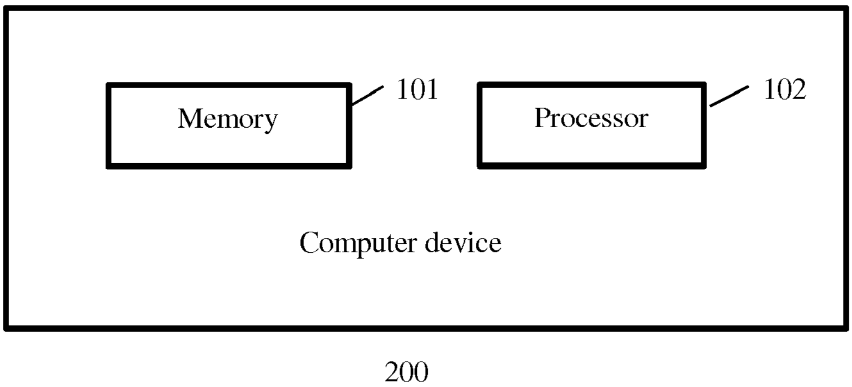

Europäisches Patentamt
European Patent Office
Office européen des brevets

# EUROPEAN SEARCH REPORT

Application Number
EP 19 19 5034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y<br>A | WO 2017/009348 A1 (COVESTRO DEUTSCHLAND AG [DE]; COVESTRO POLYMERS CO LTD [CN]) 19 January 2017 (2017-01-19)<br>* page 14, paragraphs 2,3 *<br>----- | 11,12<br><br><br>1-5,7<br>13-16 | INV.<br>B29C70/48<br>C08G18/67<br>C08J5/04 |
| Y | GB 2 437 644 A (UNIV READING [GB]) 31 October 2007 (2007-10-31)<br>* "Indicating function Polymer composition 3 Polymer composition 3 was allowed to equilibrate under ambient laboratory conditions and a colour change from blue-green to yellow-green was observed. Thus cobalt chloride can be used as an indicator that the polymer composition is adsorbing moisture.</p><br><p>Polymer composition 4 Polymer composition 4 was allowed to. equilibrate under ambient laboratory conditions and a colour change from blue to pink was observed. Thus the indicating silica gel can be used as an indicator that the polymer composition is adsorbing moisture.</p><br><p>Polymer composition 7 Polymer composition 7 was allowed to equilibrate under ambient laboratory conditions and a colour change from blue-green to yellow-green was observed. Thus the cobalt chloride can be used as an indicator that the polymer composition is adsorbing moisture.";<br>page 15, line 23 - page 16, line 8 *<br>----- | 1-5,7 | TECHNICAL FIELDS SEARCHED (IPC)<br>B29C |
| A | US 10 343 353 B2 (BOEING CO [US]) 9 July 2019 (2019-07-09)<br>* column 7, paragraph 63 - column 8, paragraph 13; figure 2 *<br>-----<br>-/-- | 6 | |

The present search report has been drawn up for all claims

| Place of search | Date of completion of the search | Examiner |
|---|---|---|
| The Hague | 29 June 2020 | Boone, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

7

Europäisches Patentamt
European Patent Office
Office européen des brevets

# EUROPEAN SEARCH REPORT

Application Number
EP 19 19 5034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2015/155195 A1 (BAYER MATERIALSCIENCE AG [DE] ET AL.) 15 October 2015 (2015-10-15) * page 2, paragraph 2 - page 3, paragraph 2 * ----- | 8,9 | |
| A | US 2018/030228 A1 (ZHANG MINGFU [US] ET AL) 1 February 2018 (2018-02-01) * paragraph [0047]; figures 1,2 * ----- | 10 | |
| A | WO 2015/084933 A1 (CONTINENTAL STRUCTURAL PLASTICS INC [US]) 11 June 2015 (2015-06-11) * claims 1,2; figure 3 * ----- | 10 | |
| Y | CN 109 435 258 A (LUOYANG SUNRUI WIND TURBINE BLADE CO LTD) 8 March 2019 (2019-03-08) * "The moisture-proof oil-absorbing pad of the invention is made of a color-developing moisture-absorbing agent or a compound of a color-developing moisture-absorbing agent and a conventional water-absorbing material and an oil-absorbing material." * ----- | 1-5 | TECHNICAL FIELDS SEARCHED (IPC) |
| A | EP 1 219 655 A1 (UBE INDUSTRIES [JP]) 3 July 2002 (2002-07-03) * paragraph [0091] * ----- | 1 | |

The present search report has been drawn up for all claims

| Place of search | Date of completion of the search | Examiner |
|---|---|---|
| The Hague | 29 June 2020 | Boone, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 19 19 5034

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 19 19 5034

The Search Division considers that the present European patent application does not comply with the requirements of unity of invention and relates to several inventions or groups of inventions, namely:

1. claims: 1-5, 7, 11-16

   A method for preparing a polyurethane composite by a vacuum infusion process

   ---

2. claim: 6

   A method covering a reinforcing material using a first film and a second film

   ---

3. claims: 8, 9

   A method for producing a polyurethane polymer starting from a component A, B and C

   ---

4. claim: 10

   A method using a reaction injection device, a vacuumizing device, a storage tank, a feeding line and a mixing unit

   ---

## ANNEX TO THE EUROPEAN SEARCH REPORT ON EUROPEAN PATENT APPLICATION NO.

EP 19 19 5034

This annex lists the patent family members relating to the patent documents cited in the above-mentioned European search report.
The members are as contained in the European Patent Office EDP file on
The European Patent Office is in no way liable for these particulars which are merely given for the purpose of information.

29-06-2020

| Patent document cited in search report | Publication date | Patent family member(s) | Publication date |
|---|---|---|---|
| WO 2017009348 A1 | 19-01-2017 | CN 106335141 A | 18-01-2017 |
| | | EP 3322570 A1 | 23-05-2018 |
| | | US 2018200924 A1 | 19-07-2018 |
| | | WO 2017009348 A1 | 19-01-2017 |
| GB 2437644 A | 31-10-2007 | GB 2437644 A | 31-10-2007 |
| | | WO 2007122412 A1 | 01-11-2007 |
| US 10343353 B2 | 09-07-2019 | CA 2938586 A1 | 06-04-2017 |
| | | EP 3153289 A1 | 12-04-2017 |
| | | ES 2726880 T3 | 10-10-2019 |
| | | KR 20170044027 A | 24-04-2017 |
| | | PT 3153289 T | 30-05-2019 |
| | | TR 201906131 T4 | 21-05-2019 |
| | | US 2017095984 A1 | 06-04-2017 |
| WO 2015155195 A1 | 15-10-2015 | CA 2944545 A1 | 15-10-2015 |
| | | CN 104974502 A | 14-10-2015 |
| | | EP 3129422 A1 | 15-02-2017 |
| | | JP 2017513979 A | 01-06-2017 |
| | | US 2017037203 A1 | 09-02-2017 |
| | | US 2019225761 A1 | 25-07-2019 |
| | | WO 2015155195 A1 | 15-10-2015 |
| US 2018030228 A1 | 01-02-2018 | EP 3115399 A1 | 11-01-2017 |
| | | US 2017066888 A1 | 09-03-2017 |
| | | US 2018030228 A1 | 01-02-2018 |
| WO 2015084933 A1 | 11-06-2015 | CA 2931030 A1 | 11-06-2015 |
| | | CN 104690876 A | 10-06-2015 |
| | | EP 3077175 A1 | 12-10-2016 |
| | | US 2016288394 A1 | 06-10-2016 |
| | | WO 2015084933 A1 | 11-06-2015 |
| CN 109435258 A | 08-03-2019 | NONE | |
| EP 1219655 A1 | 03-07-2002 | DE 60102209 T2 | 03-02-2005 |
| | | EP 1219655 A1 | 03-07-2002 |
| | | ES 2217084 T3 | 01-11-2004 |
| | | US 2002123595 A1 | 05-09-2002 |
| | | US 2005143551 A1 | 30-06-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 108152276 A
- CN 103770341 B
- US 8707762 B
- US 2017003229 A1
- CN 104974502 A

### Non-patent literature cited in the description


- Makromolekulare Stoffe. **HOUBEN WEYL.** Methoden der Organischen Chemie. Georg Thieme Verlag, 1963, vol. XIV/2, 17
- **YIJUN LIU.** Handbook of Polyurethane Raw Materials and Auxiliaries. 01 April 2005
- **HOUJUN LIU.** Polyurethane Elastomer. August 2012